Europäisches Patentamt

European Patent Office

Office européen des brevets

(19)

(11) Veröffentlichungsnummer: **0 277 459 B1**

(12) # EUROPÄISCHE PATENTSCHRIFT

(45) Veröffentlichungstag der Patentschrift: **07.04.93**

(51) Int. Cl.⁵: **A61K 9/52**, A61K 9/66

(21) Anmeldenummer: **87810672.3**

(22) Anmeldetag: **16.11.87**

Die Akte enthält technische Angaben, die nach
dem Eingang der Anmeldung eingereicht wurden
und die nicht in dieser Patentschrift enthalten sind.

(54) **Arzneimittel mit verzögerter Wirkstofffreisetzung.**

(30) Priorität: **02.02.87 CH 355/87**

(43) Veröffentlichungstag der Anmeldung:
**10.08.88 Patentblatt 88/32**

(45) Bekanntmachung des Hinweises auf die
Patenterteilung:
**07.04.93 Patentblatt 93/14**

(84) Benannte Vertragsstaaten:
**AT BE DE ES FR GB GR IT LU NL SE**

(56) Entgegenhaltungen:
**EP-A- 0 001 822**
**WO-A-86/04503**
**DE-A- 1 617 845**
**US-A- 3 932 634**
**US-A- 4 681 765**

**PATENT ABSTRACTS OF JAPAN, Band 3, Nr.
114 (C-59), 21. September 1979**

(73) Patentinhaber: **Mepha AG
Dornacherstrasse 114
CH-4147 Aesch(CH)**

(72) Erfinder: **Seth, Pawan
Stallenmattenstrasse 23
CH-4104 Oberwil(CH)**

(74) Vertreter: **Frossard, Michel, Dr. et al
A. Braun, Braun, Héritier, Eschmann AG, Patentanwälte Holbeinstrasse 36-38
CH-4051 Basel (CH)**

## Beschreibung

Die vorliegende Erfindung betrifft ein Arzneimittel, welches in der Form von Hartgelatinekapseln vorliegt und seine Wirkung infolge verzögerter Freisetzung des Wirkstoffes im Organismus nachhaltig ausübt.

Es waren bereits verschiedene Arzneimittel und pharmazeutische Präparate bekannt, die als Gelatinekapseln mit verzögerter Wirkung vorliegen oder in dieser Form dargeboten werden können. So werden in der Anmeldung DE 3 400 106 pharmazeutische Zubereitungen mit gesteuerter Arzneistoff-Freisetzung beschrieben; nebst dem Arzneistoff enthalten sie ein oder mehrere physiologisch verträgliche Polymere und ein lipophiles und/oder hydrophiles Lösungsmittel oder Quellmittel für das oder die Polymeren. Die Zubereitung kann in jeder beliebigen pharmazeutischen Form vorliegen, so u.a. auch in Form von Kapseln. Die Polymeren können Polysaccharide, Proteine oder Polyolefine sein. Als lipophile Lösungs- oder Quellmittel können Paraffine, Oele und Fette, als hydrophile Lösungs- oder Quellmittel Wasser, Alkohole, Glykole und $C_1$-$C_6$-Carbonsäuren verwendet werden; in den Beispielen finden sich Glycerin, Polyethylenglykole oder flüssiges Paraffin.

Die Herstellung pharmazeutischer Präparate in der Form von Hartgelatinekapseln wird in der Anmeldung EP 1 822 beschrieben. Die starren Hüllen werden mit einem flüssigen Träger abgefüllt, welcher den Wirkstoff enthält und in der Hülle beim Abkühlen und Stehenlassen zu einer festen Masse bzw. zu einem Gel erstarrt. Als flüssiger Träger wird eine wasserlösliche Schmelzmasse mit einem Erstarrungspunkt von 30 bis 60 °C oder ein thixotropes Gel verwendet, insbesondere Macrogolether wie Polyethylenglykole, Macrogolester wie Polyoxyl-40-stearat, Polysorbate (Polyoxyethylensorbitanfettsäureester), Sorbitanester, Polyvinylakoholsaccharoseester und Polyacrylsäure (z.B. Carbopol®). Wenn der Kapselinhalt als thixotropes Gel vorliegen soll, enthält es als Geliermittel z.B. hydriertes Ricinusöl oder kolloidales Siliciumdioxid.

Gegenstand der Patentschrift FR 7 955 M sind ebenfalls Kapseln mit verzögerter Wirkung. Sie enthalten eine Lösung oder eine flüssige oder freifliessende Suspension des Wirkstoffes und einer in Wasser unlöslichen oder sich langsam auflösenden Substanz in einem wasserlöslichen oder wasserunlöslichen Träger und mit dem Zusatz eine Lösungsvermittlers oder Tensids, welches eine Emulsion vom Typus Oel-in-Wasser zu bilden vermag. Die in Wasser unlösliche Substanz bildet mit den Säften des Magendarmtraktes einen Schaum, aus welchem der Wirkstoff langsam herausdiffundiert. Es eignen sich dafür Polyvinylverbindungen, Polycarbonate, Polystryrol, Cellulosederivate, Polyacrylsäuren, Wachse, Fette, höhere Alkohole, Siliconharze usw. Als Träger können Polyethylenglykole, Alkohole, Ester, Gemische von Ricinusöl und Ethanol oder Polyethylenglykol, Gemische von Erdnussöl und Polyoxyethylenricinusöl, Gemische von Paraffinöl und Sorbitanester verwendet werden.

Die hier zusammengefassten Dokumente sind besonders repräsentativ für Stand der Technik im Umfeld der vorliegenden Erfindung. Es handelt sich bei allen drei um ganz allgemeine Vorschläge, die immer und überall anwendbar sein sollen: Sie enthalten nämlich keinerlei Beschränkungen, Bedingungen oder Auswahl in bezug auf die Natur oder Beschaffenheit der Wirkstoffe, die es in Gelatinekapseln mit verzögerter Wirkung zu bringen gilt. Insbesondere werden keine Unterschiede danach gemacht, ob es sich um wasserlösliche bzw. hydrophile oder um wasserunlösliche bzw. lipophile Wirkstoffe handelt.

Dass jedoch die physiko-chemischen Eigenschaften der Wirkstoffe bei der Auswahl der geeigneten Hilfsstoffe eine massgebliche Rolle spielen, bedarf keiner weiteren Erörterung. Es ist selbstverständlich nicht gleichgültig, ob der betreffende Wirkstoff in Wasser leicht löslich, wenig löslich oder sogar unlöslich ist und sich dafür in den Lipiden leicht auflösen lässt. Derart grundlegende Unterschiede fallen naturgemäss umso stärker ins Gewicht, je grösser die in die Kapseln einzuarbeitende Wirkstoffmenge ist, d.h. wenn der Kapselinhalt nicht bloss zu einigen Prozenten, sondern z.B. zu 20 oder 40 % aus dem Wirkstoff besteht.

Wollte man also nach den oben geschilderten Lehre Kapseln mit verzögerter Wirkung herstellen, müsste man sich entweder auf jene Wirkstoffe beschränken, deren Bearbeitung in den Beispielen dieser Dokumente experimentell belegt ist, oder aber für andere oder neue Wirkstoffe unter den verschiedenen Gruppen von Hilfsstoffen gegensätzlicher Eigenschaften in langwierigen Versuchen jene auswählen, die für den speziellen Fall geeignet sind. Als allgemeine Lehren geben die bisher vorgeschlagenen Methoden für eine beliebigen spezifischen Wirkstoff keine stoffbezogene Handhabung her; es ist damit nicht möglich, im voraus, nur aus der physikochemischen Beschaffenheit des Wirkstoffes, die zweckmässigen Hilfsstoffe zu bestimmen.

Ein schwerwiegender Nachteil hat sich zudem bei Hartgelatinekapseln gezeigt, deren Inhalt ein Glykol, insbesondere Glycerin, Propylenglykol oder ein Polyethylenglykol in beträchtlichen Mengen umfasst, wie dies bei den Ausführungsbeispielen von DE 3 400 106, EP 1 822 und FR 7955 M der Fall ist. Wegen ihrer Hygroskopizität ziehen solche Glykole Wasser aus ihrer Umgebung an, im konkreten Fall also aus der Kapselwand. Zufolge des fortschreitenden Wasserentzugs wird die Kapselwand im Laufe der Zeit spröde und brüchig. Dies dürfte mit ein Grund sein, warum sich Kapseln nach diesen Dokumenten in der pharmazeutischen Praxis nicht durchsetzen konnten.

Ein weiterer Nachteil des Verfahrens gemäss DE 3 400 106 besteht darin, dass der Wirkstoff in eine Schmelze der Hilfsstoffe eingebracht und dieserart in die Kapseln abgefüllt wird, was meistens Temperaturen von über 100 °C und sogar 180 °C oder darüber erfordert. Die Anwendung solch hoher Temperaturen ist allerdings nur bei hitzeunempfindlichen Wirkstoffen unbedenklich, andernfalls würde sie zu einer mindestens teilweisen Inaktivierung oder Zersetzung führen; zudem verfärben sich dabei die Polyethylenglykole rasch zu einer dunklen Masse. Vor allem aber erweicht, schmilzt und verformt sich die Kapselwand bekanntlich bei Temperaturen über 70 °C [D. Cadé und Mitarb., Acta Pharmaceutica Technologica 33 - (1987), 97-100]; die Abfüllung von Gelatinakapseln muss deshalb bei niedrigeren Temperaturen erfolgen.

Nun erstarren die oben erwähnten Schmelzen bei Temperaturen unter 70 °C zu klebrigen Massen, die sich in Gelatinekapseln mit den herkömmlichen Abfüllvorrichtungen überhaupt nicht abfüllen lassen; es müssten hierfür spezielle, hitzebeständige Abfüllvorrichtungen angewendet werden, die es jedoch bisher nicht gibt.

Insgesamt fehlte bis anhin eine Universallehre, die es ermöglichen würde, einen beliebigen Wirkstoff - also auch einen hitzeempfindlichen Wirkstoff - auf anderem als bloss empirischem Wege in haltbare Gelatinekapseln unter Verwendung der üblichen Abfüllvorrichtungen und Erzielung einer genauen Dosierung zu bringen.

Es wurde nun gefunden, dass man gemäss der Erfindung jeden pharmazeutischen Wirkstoff organischer Natur in Form von haltbaren Hartgelatinekapseln mit verzögerter Wirkstofffreigabe bringen kann, ohne jedoch auf die Durchführung von Reihenversuchen angewiesen zu sein. Im Gegenteil kann die zweckmässige Zusammensetzung im voraus bestimmt werden, wenn man einzig und allein auf die Wasserlöslichkeit des Wirkstoffes abstellt und sich nach dem Grad dieser Wasserlöslichkeit bei der Auswahl der Hilfsstoffe richtet; als da sind eine Substanz oder Substanzmischung von bekanntem HLB-Wert (Hydrophil-Lipohil Balance) und ein organisches Polymer.

Die erfindungsgemässen Hartgelatinekapseln sind dadurch gekennzeichnet, dass als erster wesentlicher Hilfsstoff ein nicht ionisches Tensid bzw. ein Gemisch solcher Tenside verwendet wird, welches aus Polyglykol-ethern oder/und -estern besteht und sowohl hydrophile als auch lipophile Anteile aufweist und in bezug auf den Wirkstoff derart gewählt ist, dass sein HLB-Wert (Hydrophil-Lipophil Balance)

- für einen in Wasser nicht oder schwer löslichen Wirkstoff über 16 liegt,
- für einen in Wasser wenig löslichen Wirkstoff zwischen 16 und 10 liegt,
- für einen in Wasser löslichen Wirkstoff unter 10 liegt,

und das Polymere ein organisches Polymere ist, welches mit dem Wirkstoff und dem Tensid oder Gemisch von Tensiden eine homogene Lösung oder Suspension zu bilden vermag, die bei ca. 40 bis 70 °C in frei fliessender Form vorliegt.

Die oben definierten Kapseln enthalten den Wirkstoff in Form einer Lösung (feste Lösung) oder einer homogenen Suspension.

Im folgenden wird die Erfindung ausführlich erläutert.

Für die Einteilung dem Wasserlöslichkeit kann man sich an den unten wiedergegebenen, allgemeinen Definitionen der US-Pharmacopoeia XXI (1985), Seite 1441, bzw. Römpp Chemie Lexikon, 9. Aufl. G. Thieme Verlag, Stuttgart Bd.3, H-L, 1990, halten:

| Bezeichnung der Löslichkeit | Teile Lösungsmittel nötig für 1 Teil **Gelöstes** |
|---|---|
| Sehr löslich | weniger als 1 |
| **Leicht** löslich | von 1 bis 10 |
| Löslich | von 10 bis 30 |
| **Wenig** löslich | von 30 bis 100 |
| **Schwer** löslich | von 100 bis 1000 |
| Sehr **schwer** löslich | von 1000 bis 10 000 |
| Praktisch unlöslich oder unlöslich | 10 000 und mehr. |

Zur Vereinfachung werden hier nur die Begriffe löslich, wenig löslich und unlöslich verwendet; es ist aber klar, dass man sich auch der feineren Einteilung bedienen und den HLB-Wert des Tensids entsprechend auswählen kann.

Tenside werden im allgemeinen durch den HLB-Wert (Hydrophil-Lipophil Balance) gekennzeichnet. Ein Tensid von hohem HLB-Wert - etwa 12 bis 20 - ist hydrophil, ein Tensid von niedrigem, etwa unter 7 liegendem HLB-Wert ist lipophil.

Bei den Tensiden einer und derselben Reihe unter den veresteren Polyethylenglykolen, z.B. den Polyethylenglykol-distearaten oder -mono- oder -dipalmitostearaten, hängt der HLB-Wert vom Molekulargewicht der Molekel ab. Ganz allgemein gilt, dass der HLB-Wert steigt, wenn das Molekulargewicht grösser wird. So hat z.B. das Polyethylenglykol 400-palmitorstearat mit dem HLB-Wert 11 bis 12 einen stärker hydrophilen Charakter als das Polyethylenglykol 300-distearat mit dem HLB-Wert 9 bis 10.

Die HLB-Werte der Tenside verhalten sich additiv, so dass man den angestrebten HLB-Wert auch durch Vermischung zweier Tenside von verschiedenen HLB-Werten erreichen kann; massgeblich ist nämlich der HLB-Wert des Systems und seine Mischbarkeit mit dem organischen Polymeren unter Bildung einer flüssigen Masse. Für ein Gemisch von zwei verschiedenen Tensiden lässt sich der HLB-Endwert folgendermassen berechnen:

$$\text{HLB-Endwert} = \frac{X \cdot \text{HLB}(1) + Y \cdot \text{HLB}(2)}{X + Y}$$

X, Y :     Anteil des einen bzw. anderen Tensids im Gemisch
HLB(1):     HLB-Wert des ersten Tensids
HLB(2):     HLB-Wert des zweiten Tensids

Die als nicht ionische Tenside zu verwendenden Polyglykolderivate können u.a. Kondensationsprodukte von hydrophoben Verbindungen, wie Fettsäuren oder Fettalkohole oder Sorbitan-fettsäureester, mit Polyoxyethylenglykolen sein; diese Kondensationsprodukte werden durch Oxyethylierung der erwähnten hydrophoben Verbindungen nach bekannten Verfahren hergestellt. Die Verfahren selbst und die erhaltenen Produkte werden insbesondere in "Ullmanns Encyklopädie der technischen Chemie", 4. Auflage, Band 22, Seite 488 und folgende (Verlag Chemie GmbH, Weinheim BRD 1982) beschrieben.

Geeignete Beispiele der oben erwähnten Polyoxyethylenglykol-ester und -ether sind das Polyoxyethylenglkyol 400-palmitostearat mit HLB-Wert 11 bis 12 und die entsprechenden Verbindungen von Molekulargewicht 4000 und 1500 mit HLB-Wert über 19 bzw. 17, ferner das Polyoxyethylenglykol 300-distearat mit HLB-Wert 9 bis 10, das Laurat, Palmitat, Stearat und Oleat von durch 4 Mol Polyoxyethylenglykol

verethertem Sorbitan (zum Beispiel die Polysorbate, Tween® der ICI America Inc., Atlas Chemical Division, Wilmington DE, USA usw.) und die Polyoxyethylenglykolether des Laurylalkohols, des Cetylalkohols, des Stearylalkohols und des Oleylalkohols (zum Beispiel das Produkt Brij® der Atlas Chemie GmbH, Essen BRD). Bevorzugte nicht ionische Tenside sind die Polyoxyethylenglykol-fettsäureester.

Das organische Polymere wird nach dem verwendeten nicht ionischen Tensid derart ausgewählt, dass es mit diesem und dem Wirkstoff eine homogene, bei ca. 50 bis 60°C frei fliessende Masse bildet. Dieses Polymere kann insbesondere Methylpolyacrylat, Methylpolymeracylat, zum Beispiel die Handelsprodukte Eudragit® S 100 oder RS PM von Röhm Pharma GmbH (Darmstadt, BRD), oder Ethylcellulose oder Methylcellulose sein. Man kann auch eine Mischung von zwei oder mehrere dieser Verbindungen verwenden, sofern die rheologischen Eigenschaften der gebildeten Masse aufrechterhalten bleiben. Bekanntlich sind die genannten organischen Polymeren dem enzymatischen biologischen Abbau nicht zugänglich.

Gemäss einer besonders vorteilhaften Ausführungsform der Erfindung stellt der Wirkstoff ca. 1 bis 60 Gew.-% des Kapselinhalts, das Tensid ca. 30 bis 90 Gew.-% und das Polymere ca. 1 bis 45 Gew.-%dar.

Je nach der Natur des verwendeten Wirkstoffes kann es angebracht sein, der als Kapselinhalt vorgesehenen Masse ein Antioxydations- oder Stabilisierungsmittel zuzusetzen, zum Beispiel das tert.Butyl-4-methoxyphenol (BHA), das 2,6-Di-tert.butyl-4-methylphenol (Butylhydroxytoluol, BHT), das Octylgallat, das Dodecylgallat, die Milchsäure oder ihr Natriumsalz, der Methyl-, Ethyl-, Propyl- oder Butylester der 4-Hydroxybenzoesäure oder die entsprechenden Natriumsalze (Methyl-, Ethyl-, Propyl- und Butylparaben) usw.

Um die Viskosität der Masse zu erhöhen und dadurch der Bildung und Aufrechterhaltung einer homogenen Suspension wirksam beizutragen, kann sich der Zusatz eines Verdickungsmittels vorteilhaft erweisen; es können davon etwa bis zu 10 Gew.-% des Kapselinhalts zugesetzt werden. Das Verdickungsmittel kann zum Beispiel eine anorganische Substanz wie hochdisperses Siliciumdioxid (beispielsweise das Produkt Aerosil® der Degussa AG, Frankfurt am Main BRD), oder eine organische Substanz wie Agar-agar, Akaziengummi (Gummi arabicum), Tragantgummi, Pektine, Stärke, Dextrine, Celluloseether usw. sein.

Gewünschtenfalls kann man die mechanische Festigkeit der gebildeten Matrix durch Zugabe eines geeigneten Mittels erhöhen. Als solches kann man zum Beispiel Lactose verwenden und der Masse in einem Gewichtsverhältnis von bis zu 60 % zusetzen.

Der pharmazeutische Wirkstoff, den man dieserart in Form einer Hartgelatinekapsel bringen kann, kann sein u.a.: ein Alpharezeptorenblocker wie Dihydroergotamin und die hydrierten Derivate der Secalealkaloide, das Tolazolin, das Phentolamin; ein Betarezeptorenblocker wie Propranolol, Acebutolol, Oxyprenolol, Pindolol; ein Sympathikomimeticum, wie Dopamin und die Amphetamine; ein Analgetikum wie Glafenin, Acetylsalicylsäure, Phenacetin, Aminophenazon; ein Aldosteron-Antagonist wie Spironolacton; ein Mittel gegen die Blutplättchenagglutination wie Ticlopidin; ein Mittel gegen Angina pectoris wie Nitroglycerin, Isosorbiddinitrat; ein Mittel gegen Asthma wie Theophyllin und seine Derivate; ein Antidepressivum wie Imipramin, Desipramin, Amineptin; ein Antihistaminicum wie Pheniramin, Thenalidin, Thenyldiamin, Ketotiphen, Cyproheptadin, Azatadin; ein Antihypertensivum wie Methyldopa, Prazosin; ein Antiphlogisticum wie Ibuprofen, Naproxen, Diclofenac, Indometacin, Phenylbutazon, Oxyphenbutazon, Mefenaminsäure; ein Antiparkinsonmittel wie Trihexyphenidin, Orphenadrin, Ethopropazine; ein Tranquilizer wie Meprobamat, Lorazepam, Diazepam, Chlordiazepoxid; ein Bronchodilatator und Antiasthmaticum wie Isoprenalin, Salbutamol; ein Diureticum wie Chlorothiazid, Hydrochlorothiazid, Furosemid, Triamteren; ein Calciumantagonist wie Nifedipin, Diltiazem, Verapamil, Bepridil, Perhexilin, Prenylamin; ein Neurolepticum wie die Phenothiazin-, Butyrophenon-, Thioxanthen- und substituierten Benzamidderivate usw.

Erfindungsgemäss werden die beschriebenen Kapseln hergestellt, indem man das nicht ionische Tensid oder das Gemisch solcher Tenside auf eine über dessen Schmelzpunkt oder Erweichungspunkt liegende Temperatur erhitzt, der geschmolzenen Masse unter Beibehaltung der bereits angegebenen Temperaturen und unter Rühren bis zur Bildung einer homogenen flüssigen Masse zuerst das oder die organischen Polymeren und danach den Wirkstoff zumischt, die in der Masse verbleibende Luft entfernt und Hartgelatinekapseln mit der Menge flüssiger Masse abfüllt, welche der für eine Kapsel vorgesehenen Wirkstoffdosis entspricht. Nach Abschluss der Abfüllung erstarrt die flüssige Masse zunehmend zu einer festen Masse, ob durch Stehenlassen bei Raumtemperatur oder durch Abkühlen.

Gemäss der Erfindung werden also erstmals Hartgelatinekapseln zur Verfügung gestellt, welche durch regelmässiges Abfliessen und dauerhafte Homogenität der Zubereitung eine einwandfreie Herstellung in industriellem Massstabe ermöglichen, während der Lagerung nicht spröde und brüchig werden und den Wirkstoff in beträchtlichen Mengen enthalten und im Organismus über eine längere Zeitdauer freisetzen.

Wie schon allgemein bekannt war, wird ein in Wasser schwer löslicher Wirkstoff aus seiner Mischung mit einer hydrophilen Substanz sehr rasch freigesetzt, wenn die Mischung mit Wasser oder einem wässrigen Medium in Kontakt gebracht wird; als Beispiel sei eine Mischung von Ibuprofen und eines Polyethylenglykols (PEG) genannt. Aus einer Mischung mit einer lipophilen Substanz hingegen wird derselbe Wirkstoff nur langsam in Freiheit gesetzt.

Ueberraschenderweise wurde nun gefunden, dass die Art und Weise der Freisetzung sich völlig ändert und sich in ihr Gegenteil verkehrt, wenn der Mischung von wasserunlöslichem oder schwer löslichem Wirkstoff und hydrophilem Tensid ein mit beiden zusammen mischbares organisches Polymere zugesetzt wird.

Beispielsweise sieht man in der Tabelle 1 die Freisetzung des schwer löslichen Wirkstoffes Ibuprofen aus einer Mischung mit dem sehr hydrophilen PEG-1500-stearat bzw. dem weniger hydrophilen PEG-300-stearat: im ersten Fall werden 97 % in 0,25 Stunden, im zweiten Fall 98,1 % in 1 Stunde freigesetzt. Wird nun den beiden Mischungen das Polymere Eudragit® S 100 zugesetzt, kann man eine eindrückliche Verzögerung der Freisetzung beobachten: es werden 84,8 % in 8 Stunden bzw. 91,4 % in 2 Stunden freigesetzt. Bei dem stärker hydrophilen Tensid (HLB = 17) ist die Verzögerung, d.h. die Umkehrung des Freisetzungsvorgangs, deutlich stärker als beim weniger hydrophilen (HLB = 9 bis 10).

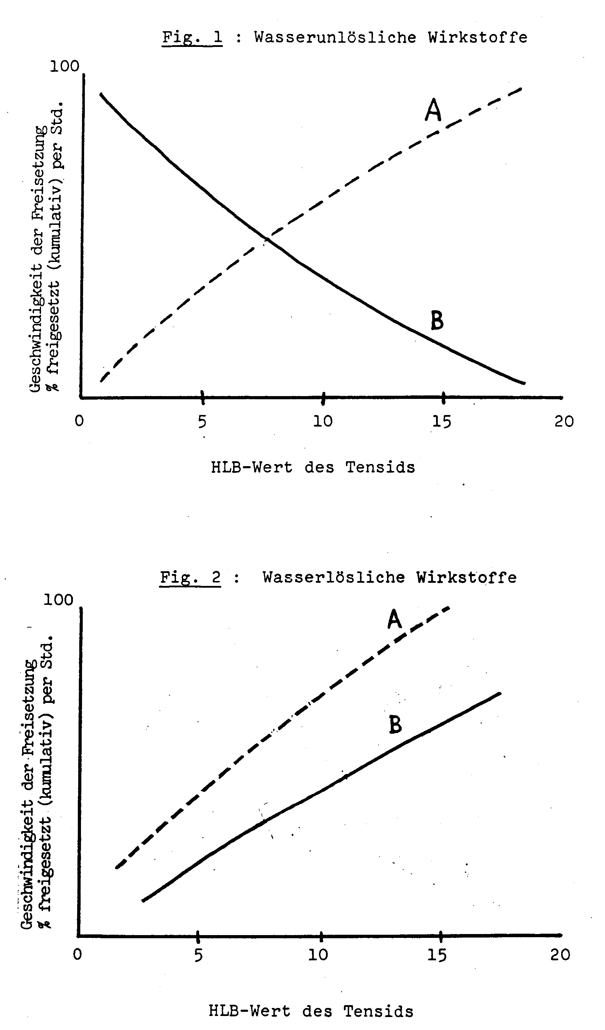

Diese Verhältnisse und ihre Umkehrung durch Zugabe eines organischen Polymeren werden durch die Figur 1 für Wirkstoffe veranschaulicht, welche in Wasser schwer löslich oder unlöslich sind. Die Kurven A und B stellen die Geschwindigkeit der Wirkstoff-Freisetzung für ein Tensid von bestimmtem HLB-Wert in Abwesenheit (A) bzw. in Gegenwart (B) eines organischen Polymeren dar. Ausgedrückt wird diese Geschwindigkeit als die Gesamtmenge Wirkstoff in Gew.-% vom Anfangsgehalt, welche nach jeder Zeiteinheit freigesetzt worden ist (Gew.-% per Std.).

| Tabelle 1 | Zum Vergleich | Beispiel 1 | Zum Vergleich | Beispiel 2 |
|---|---|---|---|---|
| Ibuprofen | 60,0 | 60,0 | 60,0 | 60,0 |
| Stearat 1500 | 40,0 } HLB = 17 | 37,4 } HLB = 17 | - } HLB = 9-10 | - } HLB = 9-10 |
| Stearat 300 | - | - | 40,0 | 37,4 |
| Eudragit S 100 | - | 2,6 | - | 2,6 |
| Zeit in Stunden | Freigesetzte Wirkstoffmenge, kumulative Werte in Gew.-% | | | |
| 0 | 0 | 0 | 0 | 0 |
| 0,1 | 20,8 | - | 8,3 | 7,2 |
| 0,25 | 97 | - | 33,2 | 22,5 |
| 0,5 | - | - | 69,9 | 43,7 |
| 1 | - | 20,3 | 98,1 | 74,5 |
| 2 | - | 34,2 | - | 91,4 |
| 4 | | 57,8 | | - |
| 6 | | 69,9 | | - |
| 8 | | 84,8 | | - |

Eine entsprechende Umkehrung der Verhältnisse findet bei wasserlöslichen Wirkstoffen nicht statt. Aus einer Mischung mit einem Tensid werden sie rasch bzw. relativ rasch freigesetzt; die Geschwindigkeit der Freisetzung nimmt mit dem hydrophilen Charakter des Tensids zu. Aus der Mischung mit einem hydrophoben Tensid werden sie also vergleichsweise langsamer freigesetzt. Nun bewirkt der Zusatz eines organischen Polymeren in jedem Fall eine deutliche Verzögerung der Freisetzung.

Dies wird am Beispiel des sehr löslichen Diltiazem·HCl in der Tabelle 2 klar gemacht. In Abwesenheit eines Polymeren werden 92,5 % in 1,5 Stunden freigesetzt; der Zusatz von Ethylcellulose verzögert die Freisetzung beträchtlich, wie aus den Beispielen 3, 4 und 5 hervorgeht. Beim Vergleich dieser Beispiele untereinander sieht man zudem, dass die Retardwirkung umso ausgeprägter ist, je stärker hydrophob das Tensidgemisch ist: bei HLB = 10,5 werden vom Wirkstoff 68,5 % in 6 Stunden, bei HLB = 11,5 hingegen 84,5 % in 3 Stunden freigesetzt.

Dieser Sachverhalt wird durch die Figur 2 für Wirkstoffe veranschaulicht, welche in Wasser löslich sind. Die Kurven A und B stellen die Geschwindigkeit der Wirkstoff-Freisetzung für ein Tensid von bestimmtem HLB-Wert in Abwesenheit (A) bzw. in Gegenwart (B) eines organischen Polymeren dar.

**Tabelle 2**

|  | Zum Vergleich | Beispiel 3 | Beispiel 4 | Beispiel 5 |
|---|---|---|---|---|
| Diltiazem·HCl | 21,0 | 21,0 | 21,0 | 21,0 |
| Stearat 400 | 55,3 } HBL = 10,9 | 69,0 } HLB ca. 11,5 | 48,3 } HLB ca. 10,9 | 34,5 } HLB ca. 10,5 |
| Stearat 300 | 23,7 | - | 20,7 | 34,5 |
| Ethylcellulose | - | 10,0 | 10,0 | 10,0 |

| Zeit in Stunden | Freigesetzte Wirkstoffmenge, kumulative Werte in Gew.-% | | | |
|---|---|---|---|---|
|  | Zum Vergleich | Beispiel 3 | Beispiel 4 | Beispiel 5 |
| 0 | 0 | 0 | 0 | 0 |
| 0,5 | 44,4 | 17,5 | 11,5 | 12,1 |
| 1 | 77,3 | 28,5 | 17,6 | 20,5 |
| 1,5 | 92,5 | - | - | - |
| 2 | - | 54,1 | 30,0 | 34,1 |
| 3 | - | 84,5 | 48,4 | 44,6 |
| 4 | - | - | 66,7 | 54,7 |
| 5 | - | - | 82,4 | 62,8 |
| 6 | - | - | 95,1 | 68,5 |

8

Ferner hat es sich als möglich erwiesen, den Grad der Verzögerung nicht nur durch die Auswahl des Tensids oder Tensidgemisches, sondern auch durch Aenderung des Anteils an organischem Polymeren in gewissen Grenzen zu steuern. Der Tabelle 3 kann man entnehmen, dass die Herabsetzung des Anteils von Eudragit® S 100 auf die Hälfte eine beträchtliche Herabsetzung der Retardwirkung zur Folge hat: die freigesetzte Wirkstoffmenge geht von 84,8 % in 8 Stunden auf ca. 100 % in 4 Stunden zurück.

Die Aenderung des Anteils an organischem Polymeren wirkt sich auf die Verzögerung der Wirkstofffreisetzung allerdings nur in gewissen Grenzen aus. Aus der Tabelle 4 wird ersichtlich, dass eine Steigerung des Polymerenanteils über einen gewissen Wert hinaus keine weitere Zunahme der Verzögerung bewirkt oder gar nachteilig sein kann. In der Zusammensetzung dürfte daher der Anteil der organischen Polymeren vorzugsweise von 2 bis 3 % als untere Grenze bis etwa 20 % als obere Grenze betragen.

Tabelle 3

| | Zum Vergleich | Beispiel 1 | Beispiel 6 |
|---|---|---|---|
| Ibuprofen | 60,0 | 60,0 | 60,0 |
| Stearat 1500 | 40,0 | 37,4 | · 38,68 |
| Eudragit S 100 | - | 2,6 | 1,32 |

| Zeit in Stunden | Freigesetzte Wirkstoffmenge, kumulative Werte in Gew.-% | | |
|---|---|---|---|
| 0 | 0 | 0 | 0 |
| 0,1 | 20,8 | - | - |
| 0,25 | 97 | - | - |
| 1 | - | 20,3 | 29,32 |
| 2 | - | 34,2 | 59,6 |
| 4 | | 57,8 | 98,1 |
| 6 | | 69,9 | 100,5 |
| 8 | | 84,8 | 101,0 |

Tabelle 4

| | Zum Vergleich | Beispiel 7 | Beispiel 8 |
|---|---|---|---|
| Verapamil·HCl | 25,0 | 25,0 | 25,0 |
| Stearat 1500 | 75,0 | 67,0 | 62,0 |
| Eudragit S 100 | - | 8,0 | 13,0 |

| Zeit in Stunden | Freigesetzte Wirkstoffmenge, kumulative Werte in Gew.-% | | |
|---|---|---|---|
| 0 | 0 | 0 | 0 |
| 0,1 | 39,8 | - | - |
| 0,5 | 98,3 | - | - |
| 1 | - | 26,0 | 33,9 |
| 2 | - | 41,5 | 46,5 |
| 4 | | 61,0 | 67,5 |
| 6 | | 75,0 | 85,9 |
| 8 | | 85,0 | 95,8 |

Im allgemeinen beobachtet man eine raschere Freisetzung des Wirkstoffes, wenn seine Konzentration in einer pharmazeutischen Zubereitung erhöht wird. Wird nun die Konzentration oder der Anteil eines schwer löslichen oder unlöslichen Wirkstoffes in den erfindungsgemässen Kapseln erhöht, so wird im Gegenteil, völlig wider Erwarten eine Verlangsamung der Wirkstofffreisetzung beobachtet. Dies geht deutlich aus der Tabelle 5 hervor: bei konstant gehaltenem Anteil an organischem Polymeren und Steigerung des Wirkstoff-

10

anteils von 20 % auf 40 % und 60 % nimmt die Verzögerung der Freisetzung von 54,15 % auf 30,09 % und schliesslich 20,88 % - jeweils in 8 Stunden - zu. Diese erstaunliche Umkehrung der bisher beobachteten Verhältnisse gilt allerdings nur für unlösliche oder schwer lösliche Wirkstoffe.

Tabelle 5

| | Zum Vergleich | Beispiel 9 | Beispiel 10 | Beispiel 11 |
|---|---|---|---|---|
| Ibuprofen | 60,0 | 20,0 | 40,0 | 60,0 |
| Stearat 4000 | 40,0 | 77,0 | 57,0 | 37,0 |
| Eudragit S 100 | - | 3,0 | 3,0 | 3,0 |

Freigesetzte Wirkstoffmenge, kumulative Werte in Gew.-%

| Zeit in Stunden | Zum Vergleich | Beispiel 9 | Beispiel 10 | Beispiel 11 |
|---|---|---|---|---|
| 0 | 0 | 0 | 0 | 0 |
| 0,1 | 18,0 | - | - | - |
| 0,25 | 78,5 | - | - | - |
| 1 | 98,9 | 25,46 | 12,1 | 8,2 |
| 2 | - | 39,38 | 16,67 | 11,81 |
| 4 | - | 45,79 | 21,95 | 15,69 |
| 6 | | 51,1 | 26,34 | 18,7 |
| 8 | | 54,15 | 30,09 | 20,88 |

11

Allgemeine Herstellungsvorschrift

In einem heizbaren Gefäss wird das nicht ionische Tensid oder Gemisch solcher Tenside vorgelegt und durch Erhitzen auf ca. 50 °C in geschmolzenen Zustand gebracht. Der geschmolzenen Masse wird das organische Polymere zugegeben und es wird bis zur vollständigen Auflösung des organischen Polymeren bzw. bis zur Bildung einer einheitlichen Suspension gerührt. Danach wird der Wirkstoff zugesetzt, bis zur Bildung einer einheitlichen Suspension gerührt und allenfalls eingeschlossene Luft durch Stehenlassen der Masse in einer Vakuumkammer entfernt. Sämtliche Verfahrensmassnahmen werden bei einer Temperatur von ca. 50 °C durchgeführt. Schliesslich wird die berechnete Menge der erhaltenen Masse in Hargelatinekapseln abgefüllt.

Im Beispiel 12 enthält die Masse das Isosorbid-dinitrat nicht als solches, weil die Handhabung dieses Wirkstoffes mit einer Explosionsgefahr einhergeht. Deshalb wird er in der Regel in Form eines Gemisches mit einem inerten Verdünnungsmittel verwendet, im vorliegenden Fall als Gemisch (20:30) mit Lactose. Dieses Gemisch wird also, an Stelle des Isosorbid-dinitrats selbst, der durchsichtigen geschmolzenen Masse zugegeben, welche nach Zugabe von Eudragit S 100 erhalten wird.

Beispiel 12

Es werden 10'000 Kapseln zu je 60 mg Isosorbiddinitrat ausgehend von den folgenden Stoffen hergestellt; in Klammern steht das Gewichtsverhältnis der betreffenden Komponente in der Zubereitung:

```
Stearat 1500                    2,090 kg    (55 %)
Eudragit S 100                  0,190 kg    ( 5 %)
Isosorbid-dinitrat/Lactose      1,520 kg    (40 %)
        (40:60)
```

In einem mit einer Rühvorrichtung und einem Vakuumanschluss versehenen, ummantelten Kolben werden genau 2,090 kg Stearat 1500 vorgelegt und der Kolben wird auf 55 °C mittels eines durch ein Thermostat gesteuerten Wasserkreislaufserhitzt und bei dieser Temperatur gehalten. Als alles Stearat 1500 geschmolzen ist, wird der Rührer bei einer Geschwindigkeit von 15 Umdrehungen/Minute in Gang gesetzt und es werden genau 190 g Eudragit S 100 zugegeben. Unter Beibehaltung der Temperatur auf 55 °C wird dem Kolben Vakuum angelegt und es wird weitergerührt bis das Eudragit S 100 im Stearat 1500 vollständig aufgelöst ist und keine Luftblasen mehr in der Lösung sichtbar sind.

Von dem Gemisch Isosorbid-dinitrat/Lactose im Gewichtsverhältnis 40:60 werden genau 1,520 kg gewogen und in die vorher erhaltene Lösung zugegeben. Die Temperatur des Kolbens wird auf 55 °C gehalten und es wird unter Vakuum während einer Stunde bei 15 Umdrehungen/Minute gerührt. Wenn die entstandene Mischung dabei Luftblasen oder zuviel Schaum zu bilden scheint, wird weiteren bei 55 °C und unter Vakuum gerührt, bis alle Luft entfernt worden ist. Die Mischung wird dann in den Fülltrichter einer Abfüllvorrichtung für Hartgelatinekapseln (z.B. Model HK-400 der Firma Robert Bosch GmbH, Waiblingen/BRD) gegeben und es werden je 380 mg geschmolzene Masse in Kapseln der Grösse 1 abgefüllt.

Im Auflösungstest zeigen die erhaltenen Kapseln eine bemerkenswerte Verzögerung der Wirkstoff-Freisetzung wie aus der folgenden Tabelle hervorgeht.

## Tabelle 6

| Zeit in Stunden | Freigesetzte Isosorbid-dinitrat-Menge kumulative Werte in Gew.-% |
|---|---|
| 0,5 | 9,2 |
| 1,0 | 17,5 |
| 2,0 | 20,5 |
| 4,0 | 27,9 |
| 6,0 | 35,5 |
| 8,0 | 43,8 |

Bestimmung der Freisetzungsgeschwindigkeit

Für jedes Beispiel ist die Geschwindigkeit gemessen worden, mit welcher der Wirkstoff aus der Kapsel in Wasser freigesetzt wird. Hierfür wurde die übliche Auflösungsmethode angewendet, die unter der Bezeichnung "Dissolution Paddle Apparatus" in der US-Pharmacopoeia XX (1979) auf Seite 959 beschrieben wird.

Zu diesem Zweck wird nach festen Zeitspannen, insbesondere nach 1, 2, 4, 6 und 8 Stunden, die gesamte freigesetzte Wirkstoffmenge (kumulative Werte) bestimmt und diese Menge in Gew.-% der in der Kapsel enthaltenen Wirkstoffmenge ausgedrückt. Die erhaltenen kumulativen Werte werden in den Tabellen angegeben.

In den Beispielen bedeutet die Abkürzung
- Stearat 4000 das Palmitostearat von Polyoxyethylenglykol-4000, eine sehr hydrophile Verbindung, HLB-Wert über 19
- Stearat 1500 das Palmitostearat von Polyoxyethylenglykol-1500, HLB-Wert 17
- Stearat 400 das Palmitostearat eines Polyoxyethylenglykols von kleinerem Molekulargewicht, deutlich weniger hydrophil, HLB-Wert 11 bis 12
- Stearat 300 das Distearat von Polyoxyethylenglykol-300, noch weniger hydrophil, HLB-Wert 9 bis 10
- Eudragit® S 100 ein aus Methyacrylsäure und Methacrylsäuremethylester bestehendes Acrylharz (Hersteller: Röhm Pharma GmbH, Darmstadt/BRD)
- Eudragit® RS PM ein Gemisch aus Acrylsäureester- und Methacrylsäureester-Copolymeren mit geringem Gehalt an quartären Ammoniumgruppen, enthält 0,5 % Talkum; als Pulvermasse mit einer Korngrösse unter 0,315 mm für mindestens 90 % und unter 1,0 mm für mindestens 99 % geliefert (Hersteller: Röhm Pharma GmbH, Darmstadt/BRD)
- Ibuprofen die 2-(4-Isobutylphenyl)-propionsäure
- Diltiazem•HCl das 3-Acetoxy-5-(2-dimethylaminoethyl)-2,3-dihydro-2-(4-methoxyphenyl)-1,5-benzothiazepin-4(5H)-on-hydrochlorid
- Verapamil•HCl das 5-N-(3,4-Dimethoxyphenethyl)-N-methylamino-2-(3,4-dimethoxyphenyl)-2-isopropylvaleronitrilhydrochlorid

Zusätzlich zum Auflösungstest wurde die Bioverfügbarkeit von repräsentativen erfindungsgemässen Kapseln im Vergleich mit jenen eines Handelspräparates derselben Klasse untersucht; es sind hierfür Kapseln der folgenden Zusammensetzung verwendet worden.

Verapamil-Kapseln 2 KL 917

| | | |
|---|---|---|
| Stearat 400 | 79,7 mg | 18,6 % |
| Stearat 300 | 186,0 | 43,4 |
| Ethylcellulose N 10 | 42,8 | 10,0 |
| Verapamil·HCl | 120,0 | 28,0 |
| | 428,5 mg | 100 % |

Verapamil-Kapseln 4KL 937 (gemäss Beispiel 8)

| | | |
|---|---|---|
| Stearat 1500 | 297,6 mg | 62,0 % |
| Eudragit ®RS PM | 62,4 | 13,0 |
| Verapamil·HCl | 120,0 | 25,0 |
| | 480,0 mg | 100 % |

Die Untersuchung wurde durch Ian W. French and Associates Ltd., Markham (Ontario, Kanada) im Januar 1986 an sechs gesunden, 18 bis 40 Jahre alten männlichem Freiwilligen durchgeführt. Jeder Freiwillige bekam einmal eine Kapsel jeder der beiden Formel mit einem einwöchigen Intervall. Zur Bestimmung der Verapamil-Konzentration im Blutplasma wurden Blutproben zum Zeitpunkt 0 und 1, 2, 3, 4, 6, 8, 10, 12, 16 und 24 Stunden nach Verarbreichung der Kapsel entnommen. Die Ergebnisse der Bestimmungen werden in der folgenden Tabelle wiedergegeben.

Tabelle 7

Durchschnittliche Verapamil-Konzentration im Blutplasma

(in ng/ml)

| Zeitpunkt der Blutentnahme (in Stunden) | Kapseln 4 KL 937 (1 x 120 mg Verapamil) | Kapseln 2 KL 917 (1 x 120 mg Verapamil) | Tabletten Isoptin-Retard[*] (1 x 120 mg Isoptin) |
|---|---|---|---|
| 0 | 0 | 0 | 0 |
| 1,0 | 0 | 5,37 ± 8,71 | 1,89 ± 4,24 |
| 2,0 | 4,16 + 4,62 | 14,98 ± 5,17 | 7,26 ± 8,15 |
| 3,0 | 9,22 ± 9,01 | 18,02 ± 13,09 | 20,09 ± 21,71 |
| 4,0 | 14,02 ± 18,39 | 20,90 ± 14,38 | 27,67 ± 27,10 |
| 6,0 | 22,94 ± 19,97 | 20,66 ± 15,32 | 30,00 ± 14,70 |
| 8,0 | 21,82 ± 11,49 | 15,64 ± 14,37 | 21,70 ± 9,78 |
| 10,0 | 16,02 ± 8,47 | 15,41 ± 10,08 | 18,26 ± 8,08 |
| 12,0 | 12,48 ± 9,63 | 12,87 ± 9,02 | 12,51 ± 7,73 |
| 16,0 | 9,99 ± 7,19 | 8,86 ± 11,66 | 4,61 ± 5,52 |
| 24,0 | 3,63 ± 5,80 | 3,61 ± 5,66 | 1,31 ± 3,21 |

Pharmakokinetische Parameter

| | Kapseln 4 KL 937 | Kapseln 2 KL 917 | Tabletten Isoptin-Retard |
|---|---|---|---|
| AUC 0-24 Std. (ng·Std./ml) | 269,82 ± 154,62 | 276,81 ± 101,46 | 281,95 ± 141,77 |
| $C_{max}$ (ng/ml) | 29,34 ± 16,38 | 33,78 ± 7,19 | 38,78 ± 23,14 |
| $T_{max}$ (Std.) | 7,00 ± 3,74 | 7,17 ± 5,00 | 6,33 ± 2,94 |

AUC : (Aera Under the Curve) durch die Kurve umfasste Fläche

$C_{max}$ : maximale Konzentration im Plasma

$T_{max}$ : Zeitpunkt der maximalen Konzentration

[*]Hersteller: Knoll AG, Ludwigshafen/BRD.

Diese Ergebnisse zeigen, dass die Kapseln der zwei oben angegebenen Zusammensetzungen einander durchaus vergleichbar sind in bezug auf AUC und $C_{max}$, das heisst bezüglich der Resorption oder Bioverfügbarkeit des Wirkstoffes. Sie zeigen ferner, dass die Retardwirkung, d.h. die langsame und anhaltende Freisetzung des Wirkstoffes im Organismus, wie sie insbesondere durch einen Wert $T_{max}$ von ca. 7 Stunden zum Ausdruck kommt, durch beide Zubereitungen etwa gleich gut erbracht wird. Beim Vergleichspräparat liegt der Wert von $C_{max}$ höher und die Kurve der Verapamil-Konzentration verläuft - bei etwa gleichem AUC-Wert - steiler und auch steiler abfallend; die Retardwirkung der Isoptin-Tabeletten ist also weniger ausgeprägt.

Zusammenfassend lässt sich sagen, dass die Untersuchung der Bioverfügbarkeit nach den in der Klinik angewendeten strengen Prüfungsmethoden die Retardwirkung vollauf bestätigt hat, welche für die erfindungsgemässen Kapseln bei der Untersuchung der Verzögerung der Wirkstofffreisetzung in vitro gezeigt worden ist.

**Patentansprüche**

1.  Arzneimittel mit verzögerter Wirkstoff-Freigabe in Form von Hartgelatinekapseln, die
    a) einen Wirkstoff,
    b) mindestens eine Substanz von hydrophilem bzw. lipophilem Charakter und
    c) ein pharmazeutisch unbedenkliches Polymer
    enthalten, dadurch gekennzeichnet, dass die Komponente b) mindestens ein nicht ionisches Tensid ist, das aus Polyglykolethern und/oder Polyglykol-estern gewählt ist, dass der HLB-Wert der Komponente b) in Abhängigkeit von der Wasserlöslichkeit des Wirkstoffs derart gewählt ist, dass er
    -  für einen Wirkstoff, von dem sich 1 Teil in 100 oder mehr Teilen Wasser löst, über 16 beträgt,
    -  für einen Wirkstoff, von dem sich 1 Teil in 30 bis 100 Teilen Wasser löst, 16 bis 10 beträgt,
    -  für einen Wirkstoff, von dem sich 1 Teil in 30 oder weniger Teilen Wasser löst, unter 10 beträgt,
    und dass die Komponente c) mindestens ein organisches Polymer ist, das mit dem Wirkstoff und dem Tensid oder Tensidgemisch eine homogene Lösung oder Suspension zu bilden vermag, die unter etwa 70°C in frei fliessender Form vorliegt, wobei die Komponente c) die Freigabegeschwindigkeit des Wirkstoffs derart beeinflusst, dass sich ein Arzneimittel mit verzögerter Wirkstoff-Freigabe ergibt.

2.  Arzneimittel nach Anspruch 1, dadurch gekennzeichnet, dass das Tensid ein Polyoxyethylenglykol-fettsäureester, ein Polyoxyethylenglykol-fettalkoholether oder ein Fettsäureester von durch Polyoxyethylenglykol veräthertem Sorbitan ist.

3.  Arzneimittel nach Anspruch 2, dadurch gekennzeichnet, dass das Tensid das Palmitostearat von Polyoxyethylenglykolen der folgenden Molekulargewichte: 4000, mit HLB-Wert über 19; 1500, mit HLB-Wert 17; 400, mit HLB-Wert 11 bis 12; das Polyoxyethylenglykol-300-distearat, mit HLB-Wert 9 bis 10; das Laurat, Palmitat, Stearat oder Oleat von durch 4 Mol Polyoxyethylenglykol verethertem Sorbitan, oder ein Gemisch zweier oder mehrerer dieser Verbindungen ist.

4.  Arzneimittel nach Anspruch 2, dadurch gekennzeichnet, dass das Tensid ein Polyoxyethylenglykolether des Laurylalkohols, des Cetylalkohols, des Stearylalkohols, des Oleylalkohols oder ein Gemisch zweier oder mehrerer dieser Verbindungen ist.

5.  Arzneimittel nach einem der Ansprüche 1 bis 4, dadurch gekennzeichnet, dass das organische Polymere Methylpolyacrylat, Methylpolymethacrylat, Ethylcellulose, Methylcellulose oder ein Gemisch zweier oder mehrerer dieser Verbindungen ist.

6.  Arzneimittel nach einem der Ansprüche 1 bis 4, dadurch gekennzeichnet, dass der Wirkstoff ca. 1 bis 60 %, das Tensid ca. 30 bis 90 % und das Polymere ca. 1 bis 45 %, bezogen auf das Gewicht des Kapselinhalts, darstellt.

7.  Arzneimittel nach einem der Ansprüche 1 bis 4, dadurch gekennzeichnet, dass die Kapseln ausserdem ein Antioxidations- oder Stabilisierungsmittel enthalten.

8.  Arzneimittel nach einem der Ansprüche 1 bis 4, dadurch gekennzeichnet, dass die Kapseln ausserdem ein Verdickungsmittel enthalten.

9.  Arzneimittel nach einem der Ansprüche 1 bis 4, dadurch gekennzeichnet, dass die Kapseln ausserdem ein Mittel zur Erhöhung der mechanischen Festigkeit des Kapselinhaltes enthalten.

10. Verfahren zur Herstellung des Arzneimittels nach einem der Ansprüche 1 bis 9, dadurch gekennzeichnet, dass man das nicht ionische Tensid oder das Gemisch solcher Tenside auf eine über dessen Schmelzpunkt oder Erweichungspunkt liegenden Temperatur erhitzt, der geschmolzenen Masse unter Beibehaltung der bereits angegebenen Temperatur und unter Rühren bis zur Bildung einer homogenen flüssigen Masse zuerst das oder die organischen Polymeren und danach den Wirkstoff zumischt, die in der Masse verbleibende Luft entfernt und Hartgelatinekapseln mit der Menge flüssiger Masse abfüllt, welche der für eine Kapsel vorgesehenen Wirkstoffdosis entspricht.

**Claims**

1. A medicament with a delayed release of active ingredient in the form of hard gelatin capsules which contain
   a) an active ingredient,
   b) at least one substance of hydrophilic or lipophilic character and
   c) a pharmaceutically acceptable polymer,
   characterized in that component b) is at least one nonionic surfactant selected from polyglycol ethers and/or polyglycol esters, that the HLB value of component b) is selected, dependent on the water solubility of the active ingredient, such that it amounts to
   - more than 16 for an active ingredient, 1 part of which dissolves in 100 or more parts of water,
   - 16 to 10 for an active ingredient, 1 part of which dissolves in 30 to 100 parts of water,
   - less than 10 for an active ingredient, 1 part of which dissolves in 30 or less parts of water,
   and that component c) is at least one organic polymer which is capable of forming a homogeneous solution or suspension with the active ingredient and the surfactant or mixture of surfactants, the solution or suspension being in the free-flowing form below about 70°C, component c) influencing the rate of release of the active ingredient such that a medicament with a delayed release of active ingredient results.

2. A medicament as claimed in claim 1, characterized in that the surfactant is a polyoxyethylene glycol fatty acid ester, a polyoxyethylene glycol fatty alcohol ether or a fatty acid ester of sorbitan etherified by polyoxyethylene glycol.

3. A medicament as claimed in claim 2, characterized in that the surfactant is the palmitostearate of a polyoxyethylene glycol of the following molecular weight: 4000, with an HLB value above 19; 1500, with an HLB value of 17; or 400, with an HLB value of 11 to 12; polyoxyethylene glycol 300-distearate with an HLB value of 9 to 10; the laurate, palmitate, stearate or oleate of sorbitan etherified by 4 mols of polyoxyethylene glycol, or a mixture of two or more of these compounds.

4. A medicament as claimed in claim 2, characterized in that the surfactant is a polyoxyethylene glycol ether of lauryl alcohol, cetyl alcohol, stearyl alcohol or oleyl alcohol or a mixture of two or more of these compounds.

5. A medicament as claimed in one of claims 1 to 4, characterized in that the organic polymer is methyl polyacrylate, methyl polymethacrylate, ethylcellulose, methylcellulose or a mixture of two or more of these compounds.

6. A medicament as claimed in one of claims 1 to 4, characterized in that the active ingredient makes up about 1 to 60%, the surfactant makes up about 30 to 90% and the polymer makes up about 1 to 45% of the weight of the contents of the capsule.

7. A medicament as claimed in one of claims 1 to 4, characterized in that the capsules also contain an antioxidant or stabilizer.

8. A medicament as claimed in one of claims 1 to 4, characterized in that the capsules also contain a thickening agent.

9. A medicament as claimed in one of claims 1 to 4, characterized in that the capsules also contain an agent for increasing the mechanical strength of the contents of the capsules.

10. A process for the preparation of a medicament as claimed in one of claims 1 to 9, characterized by heating the nonionic surfactant or the mixture of such surfactants to a temperature above the melting point or softening point thereof, mixing first the organic polymer or polymers and then the active ingredient with the molten mass, while maintaining the temperature already stated and with stirring, until a homogeneous liquid composition is formed, removing the air remaining in the composition and filling hard gelatin capsules with the amount of liquid composition corresponding to the dose of active ingredient envisaged for one capsule.

**Revendications**

1. Médicament à libération retardée, sous forme de capsules de gélatine dure qui contiennent
   a) une substance active,
   b) au moins une substance de nature hydrophile ou lipophile et
   c) un polymère pharmaceutiquement acceptable,
   caractérisé en ce que le composant b) est au moins un agent tensioactif non ionique, choisi parmi les éthers de polyglycol et/ou les esters de polyglycol, la valeur HLB du composant b) est choisie en fonction de la solubilité dans l'eau de la substance active de façon telle
   - qu'elle soit supérieure à 16 pour une substance active dont une partie se dissout dans 100 parties ou plus d'eau,
   - qu'elle soit de 16 à 10 pour une substance active dont une partie se dissout dans 30 à 100 parties d'eau,
   - qu'elle soit inférieure à 10 pour une substance active dont une partie se dissout dans 30 parties ou moins d'eau,
   et que le composant c) est au moins un polymère organique capable de former avec la substance active et l'agent tensioactif ou le mélange d'agents tensioactifs une solution ou suspension homogène à l'état d'écoulement libre au-dessous d'environ 70°C, le composant c) déterminant la vitesse de libération de la substance active de manière telle qu'il en résulte un médicament à libération retardée de la substance active.

2. Médicament selon la revendication 1, caractérisé en ce que l'agent tensioactif est un ester d'acide gras de polyoxyéthylèneglycol, un éther d'alcool gras de polyoxyéthylèneglycol ou un ester d'acide gras de sorbitane éthérifié par un polyoxyéthylèneglycol.

3. Médicament selon la revendication 2, caractérisé en ce que l'agent tensioactif est le palmitostéarate des polyoxyéthylèneglycols de poids moléculaires suivants: 4 000, avec une valeur HLB supérieure à 19; 1 500, avec une valeur HLB de 17; 400, avec une valeur HLB de 11 à 12; le distéarate de polyoxyéthylèneglycol 300, avec une valeur HLB de 9 à 10; le laurate, le palmitate, le stéarate ou l'oléate de sorbitane éthérifié par 4 molécules de polyoxyéthylèneglycol ou un mélange de deux ou plusieurs de ces composés.

4. Médicament selon la revendication 2, caractérisé en ce que l'agent tensioactif est un éther de polyoxyéthylèneglycol de l'alcool laurylique, de l'alcool cétylique, de l'alcool stéarylique, de l'alcool oléylique ou un mélange de deux ou plusieurs de ces composés.

5. Médicament selon l'une quelconque des revendications 1 à 4, caractérisé en ce que le polymère organique est le polyacrylate de méthyle, le polyméthacrylate de méthyle, l'éthylcellulose, la méthylcellulose ou un mélange de deux ou plusieurs de ces composés.

6. Médicament selon l'une quelconque des revendications 1 à 4, caractérisé en ce que la substance active représente environ 1 à 60%, l'agent tensioactif environ 30 à 90% et le polymère environ 1 à 45% par rapport au poids du contenu de la capsule.

7. Médicament selon l'une quelconque des revendications 1 à 4, caractérisé en ce que les capsules contiennent, en outre, un agent antioxydant ou un agent de stabilisation.

8. Médicament selon l'une quelconque des revendications 1 à 4, caractérisé en ce que les capsules contiennent, en outre, un agent épaississant.

9. Médicament selon l'une quelconque des revendications 1 à 4, caractérisé en ce que les capsules contiennent, en outre, un agent d'amélioration de la résistance mécanique du contenu de la capsule.

**10.** Procédé de préparation du médicament selon l'une quelconque des revendications 1 à 9, caractérisé en ce que l'on chauffe l'agent tensioactif non ionique ou le mélange de tels agents tensioactifs à une température située au-dessus de leur point de fusion ou de leur point de ramolissement, on ajoute à la masse fondue, en mélangeant, en maintenant la température mentionnée ci-dessus et en agitant jusqu'à formation d'une masse liquide homogène, d'abord le ou les polymères organiques, puis la substance active, on élimine l'air demeuré dans la masse et on remplit des capsules de gélatine dure de la quantité de masse liquide qui correspond à la dose de substance active prévue pour une capsule.

Fig. 1 : Wasserunlösliche Wirkstoffe

HLB-Wert des Tensids

Fig. 2 : Wasserlösliche Wirkstoffe

HLB-Wert des Tensids